# FASCICULE DE BREVET EUROPEEN

(11) **EP 3 760 277 B1**
(45) Date de publication et mention de la délivrance du brevet: **21.09.2022**
(21) Numéro de dépôt: 20183494.2
(22) Date de dépôt: 01.07.2020
(51) Int. Cl.: A61N 1/05, A61N 1/39

(54) **SYSTÈME DE DÉFIBRILLATION CARDIAQUE IMPLANTABLE SOUS CUTANE**
SUBKUTAN IMPLANTIERBARES KARDIALES DEFIBRILLATIONSSYSTEM
SUBCUTANEOUS IMPLANTABLE CARDIAC DEFIBRILLATION SYSTEM

(30) Priorité: 05.07.2019 FR 1907532
(43) Date de publication de la demande: 06.01.2021
(73) Titulaire: Sorin CRM SAS, 92140 Clamart (FR)
(72) Inventeur: LEGAY, Thierry, 91640 Fontenay Les Briis (FR); CORDERO ALVAREZ, Rafael, 75014 Paris (FR); FEUERSTEIN, Delphine, 92100 Boulogne Billancourt (FR)
(74) Mandataire: Ungerer, Olaf

(56) Documents cités:
- WO-A1-92/17240
- WO-A1-2017/192870
- DE-A1- 19 800 697
- US-A1- 2014 330 325

## Description

La présente invention se rapporte à un système de défibrillation cardiaque implantable sous cutané.

Les défibrillateurs automatiques implantables classiques, c'est-à-dire de type transveineux, aussi abréviés ICD pour « Implantable Cardioverter-Defibrillator » en anglais, comprennent un générateur d'impulsions de défibrillation et une unité de contrôle à microprocesseur logés dans un boîtier métallique généralement implanté de manière sous-cutanée dans la poche pectorale. Ce boîtier est connecté à une ou plusieurs sondes qui sont introduites dans la veine sous-clavière où elles atteignent finalement le cœur. À l'intérieur du cœur, les extrémités distales des sondes sont fixées aux parois internes des cavités cardiaques, où elles peuvent enregistrer des électrogrammes (EGM) reflétant le fonctionnement électrophysiologique du cœur. Sur la base de ceux-ci, un traitement sous forme de défibrillation (choc de défibrillation) est administré (ou interrompu) afin de mettre fin à une tachyarythmie ventriculaire représentant un danger de mort, telle que la tachycardie ventriculaire et la fibrillation ventriculaire.

US 2014/330325 décrit un système cardiaque implantable comprenant un S-ICD (S-ICD pour « Subcutaneous- Implantable Cardioverter-Defibrillator ») et une sonde de défibrillation et une sonde de stimulation. La sonde de défibrillation comprend une électrode de défibrillation et deux électrodes.

Les éléments les plus faibles de ces défibrillateurs automatiques implantables transveineux (ainsi que des stimulateurs cardiaques et dispositifs similaires) sont les sondes intracardiaques. La fracture de sonde est d'ailleurs l'une des causes les plus courantes de dysfonctionnement du stimulateur cardiaque. L'extraction d'une sonde ICD (ou d'un stimulateur cardiaque) implantée est une procédure à forte morbidité et mortalité élevée, et est donc généralement uniquement effectuée en cas d'infection systémique grave ne pouvant pas être traitée avec des antibiotiques. Dans la majorité des cas, les sondes fracturées seront déconnectées du dispositif et laissées dans le cœur. Une nouvelle sonde est ensuite implantée à côté de l'ancienne et connectée au défibrillateur automatique implantable. Cependant, cette solution n'est possible que s'il reste encore suffisamment d'espace dans la veine, car la présence de davantage de sondes peut entraîner une occlusion veineuse. Par conséquent, l'utilisation de sondes intracardiaques n'est pas idéalement adaptée à de jeunes patients, qui pourraient avoir besoin d'une multitude de sondes au cours de leur vie.

Une solution aux problèmes associés aux sondes intracardiaques, énumérés ci-dessus, consiste à les remplacer par des sondes sous-cutanées. Ainsi, en l'absence de contact avec le cœur ou le sang, le risque d'infection systémique est éliminé et les veines ne sont plus obstruées. En outre, contrairement aux sondes intracardiaques, l'extraction des sondes sous-cutanées est moins traumatique et ne comporte pas de risque de mortalité, car les sondes sous-cutanées ne touchent pas le cœur. De ce fait, les sondes peuvent être retirées en toute sécurité en cas de fracture et remplacées par de nouvelles sondes sous-cutanées, sans risque pour le patient.

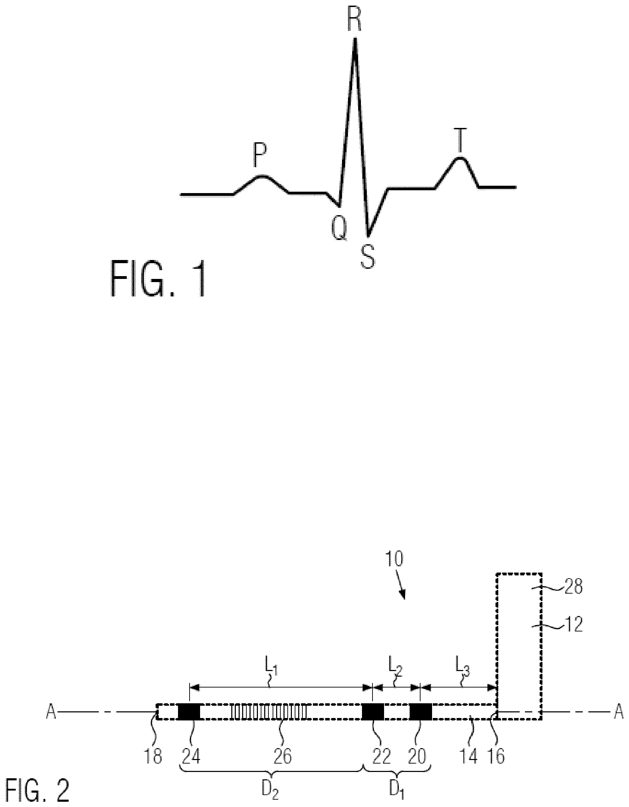

Les principaux défis des dispositifs implantables sous-cutanés sont liés à la réduction du rapport signal sur bruit des signaux enregistrés par voie sous-cutanée et à l'augmentation de l'énergie requise pour une défibrillation réussie. Les dispositifs implantables sous-cutanés enregistrent des électrocardiogrammes (ECG - la Figure 1 illustre un tracé typique représentant un ECG normal) sous-cutanés - plutôt que des EGM - qui capturent une activité électrophysiologique spatialement moyennée en champ lointain. Les ondes P et T sont plus significatives dans les ECGs sous-cutanés que dans les EGMs intracardiaques, ce qui rend plus difficile la détection des ondes R et donc le calcul des intervalles RR au moyen d'algorithmes de détection de tachyarythmie. Une interprétation erronée des signaux recueillis pourrait entraîner, par exemple dans un défibrillateur automatique implantable, un choc inapproprié, pouvant être traumatique, voire délétère pour le patient. Les sources de bruit non cardiaques, telles que les myopotentiels, peuvent également altérer les signaux sous-cutanés et interférer avec les algorithmes de détection, ce qui perturbe le traitement. De plus, les signaux sous-cutanés ont tendance à être plus sensibles aux changements de posture que les signaux intracardiaques.

Afin de pallier aux limitations susmentionnées, l'objet de la présente invention est d'améliorer la détection, notamment en terme de sensibilité et de discrimination, des signaux électrophysiologiques enregistrés par un système de défibrillation cardiaque implantable sous cutané.

L'objet de présente invention est atteint par un système de défibrillation cardiaque implantable sous cutané comprenant un boîtier; et une sonde implantable sous cutanée comprenant deux extrémités, une extrémité proximale étant connectée au boîtier et une extrémité distale étant une extrémité libre ; la sonde implantable sous cutanée comprenant au moins une électrode de défibrillation et au moins trois électrodes de détection ; la première électrode de détection et la deuxième électrode de détection formant un premier dipôle ; la troisième électrode de détection et la première électrode de détection, ou, la troisième électrode de détection et la deuxième électrode de détection, ou, le boîtier et l'une desdites électrodes de détection, formant un deuxième dipôle ; l'électrode de défibrillation étant positionnée entre la deuxième électrode de détection et la troisième électrode de détection ; le premier dipôle étant positionné entre le boîtier et l'électrode de défibrillation ; la troisième électrode étant positionnée entre l'extrémité distale libre de la sonde et l'électrode de défibrillation ; et la longueur du premier dipôle étant plus courte que la longueur du deuxième dipôle.

Le premier dipôle, de par la spécificité de son positionnement sur la sonde et de sa dimension par rapport au deuxième dipôle, permet de détecter sélectivement des signaux électrophysiologiques améliorant la détection de l'onde R et minimisant celle des ondes cardiaques P et T. Un tel agencement du premier dipôle permet ainsi d'améliorer la mesure de l'intervalle R-R. De plus, cette configuration spécifique des dipôles du système de défibrillation cardiaque implantable sous cutané permet également de réduire les bruits et artefacts, par exemple générés par la masse musculaire du patient, auxquels le premier dipôle est moins susceptible étant davantage court que le deuxième dipôle, et donc d'améliorer davantage la qualité de la détection du signal électrophysiologique, notamment en réduisant les risques de surdétection.

La présente invention, relative à un système de défibrillation cardiaque implantable sous cutané, peut être davantage améliorée grâce aux modes de réalisation suivants.

Selon un mode de réalisation de l'invention, le système de défibrillation cardiaque implantable sous cutané peut en outre comprendre un contrôleur configuré pour la détection de signaux électrophysiologiques concurremment enregistrés via le premier dipôle et le deuxième dipôle de la sonde implantable sous cutanée ; le contrôleur pouvant être configuré pour la détection de l'onde R d'un signal électrophysiologique via le premier dipôle.

Le premier dipôle, étant plus court que le deuxième dipôle, est moins exposé au risque de surdétection. En effet, la distance couverte entre les électrodes du premier dipôle est réduite comparée à celle du deuxième dipôle, ce qui diminue les risques d'altération des signaux par une source externe. Il y a, par exemple, moins de masse musculaire entre les électrodes du premier dipôle risquant d'introduire des myopotentiels. Une détection de l'onde R via le premier dipôle permet ainsi d'améliorer la qualité de la détection de l'onde R par rapport aux systèmes de défibrillation cardiaques implantables sous cutanés connus. Ainsi, la mesure de l'intervalle R-R peut être davantage améliorée.

Selon un mode de réalisation de l'invention, la longueur du premier dipôle, soit la distance entre la première électrode de détection et la deuxième électrode de détection, peut être comprise entre 5 et 50 millimètres, en particulier entre 10 et 20 millimètres ; et dont la longueur du deuxième dipôle, soit la distance entre la troisième électrode de détection et la première électrode de détection, ou, la troisième électrode de détection et la deuxième électrode de détection, ou, le boîtier et l'une desdites électrodes de détection, peut être comprise entre 50 et 400 millimètres.

Il s'avère qu'une amélioration de la qualité des signaux détectés via le premier dipôle et le deuxième dipôle est exhibée à ces longueurs spécifiques.

Selon un mode de réalisation de l'invention, la sonde implantable sous cutanée peut comprendre au moins un moyen de fixation pour fixer la sonde aux tissus d'un patient.

Le positionnement de la sonde implantable sous cutanée est alors assuré grâce au moyen de fixation, évitant ainsi un déplacement involontaire de la sonde lors d'un mouvement du patient par exemple. Un tel déplacement involontaire pourrait modifier la position du premier dipôle et du deuxième dipôle relativement au corps du patient, et affecter la qualité des signaux électrophysiologiques détectés via ces dipôles.

Selon un mode de réalisation de l'invention, le au moins un moyen de fixation de la sonde implantable sous cutanée peut être positionné entre la deuxième électrode de détection et l'électrode de défibrillation.

Ce positionnement particulier du moyen de fixation permet notamment de courber la sonde au niveau du moyen de fixation de manière à former un angle, en particulier essentiellement droit, entre les portions de la sonde qui se trouvent de part et d'autre de ce moyen de fixation.

Selon un mode de réalisation de l'invention, le au moins un moyen de fixation de la sonde implantable sous cutanée peut être positionné entre 60 et 300 millimètres de l'extrémité proximale ; et le au moins un moyen de fixation de la sonde implantable sous cutanée peut être positionné entre 50 et 400 millimètres de l'extrémité distale.

Une amélioration de la qualité des signaux détectés via le premier dipôle et le deuxième dipôle est exhibée à ces dimensions spécifiques. En effet, ces dimensions spécifiques permettent à la fois de définir le positionnement du premier dipôle et du deuxième dipôle l'un par rapport à l'autre, mais aussi par rapport à la masse musculaire d'un patient lorsque le système est implanté - le boîtier d'un tel système étant usuellement implanté au même endroit dans chaque patient, c'est-à-dire du côté gauche de la poitrine du patient selon les pratiques médicales connues.

Selon un mode de réalisation de l'invention, le au moins un moyen de fixation de la sonde implantable sous cutanée peut être une rainure sur la circonférence de la sonde implantable sous cutanée, la largeur de la rainure pouvant être dimensionnée pour y loger un fil de ligature.

Le moyen de fixation est ainsi adapté pour qu'un praticien puisse effectuer une ligature de la sonde sous cutanée à la masse musculaire du patient, et ainsi assurer le maintien et le positionnement de la sonde sous-cutanée et de ses dipôles.

Selon un mode de réalisation de l'invention, la distance entre le boîtier et la première électrode détection peut être comprise entre 40 et 300 millimètres.

Une amélioration de la qualité des signaux détectés via le premier dipôle est exhibée à cette dimension spécifique de la première électrode par rapport au boîtier, notamment car elle est adaptée pour pouvoir positionner le premier dipôle de la sonde au-dessus de l'encoche cardiaque du poumon gauche.

Selon un mode de réalisation de l'invention, le défibrillateur implantable sous cutanée peut en outre comprendre un accéléromètre ou/et un gyroscope configuré(s) pour détecter la position du patient.

En permettant la détection de la position du patient, l'interprétation des signaux électrophysiologiques détectés est davantage affinée, et donc améliorée.

Selon un mode de réalisation de l'invention, le deuxième dipôle peut être formé par la troisième électrode de détection et la deuxième électrode de détection.

Le deuxième dipôle formé par la troisième électrode de détection et la deuxième électrode de détection a une géométrie davantage adaptée à l'amélioration de la détection de signaux électrophysiologiques, en particulier lorsque la sonde est courbée de manière essentiellement perpendiculaire au niveau du moyen de fixation. En effet, un tel agencement perpendiculaire entre la deuxième électrode de détection et la troisième électrode de détection permet d'améliorer la qualité du signal détecté par le deuxième dipôle.

L'invention et ses avantages seront expliqués plus en détail dans la suite au moyen de modes de réalisation préférés et en s'appuyant notamment sur les figures d'accompagnement suivantes, dans lesquelles :
Figure 1 représente un tracé schématique d'un électrocardiogramme normal ;
Figure 2 représente une vue schématisée d'un système de défibrillation cardiaque implantable sous cutané selon la présente invention ;
Figure 3 représente une vue schématisée et par transparence du système de défibrillation cardiaque implantable sous cutané selon la présente invention implanté ;
Figure 4 représente des signaux électrophysiologiques recueillis au moyen du système de défibrillation cardiaque implantable sous cutané selon la présente invention.

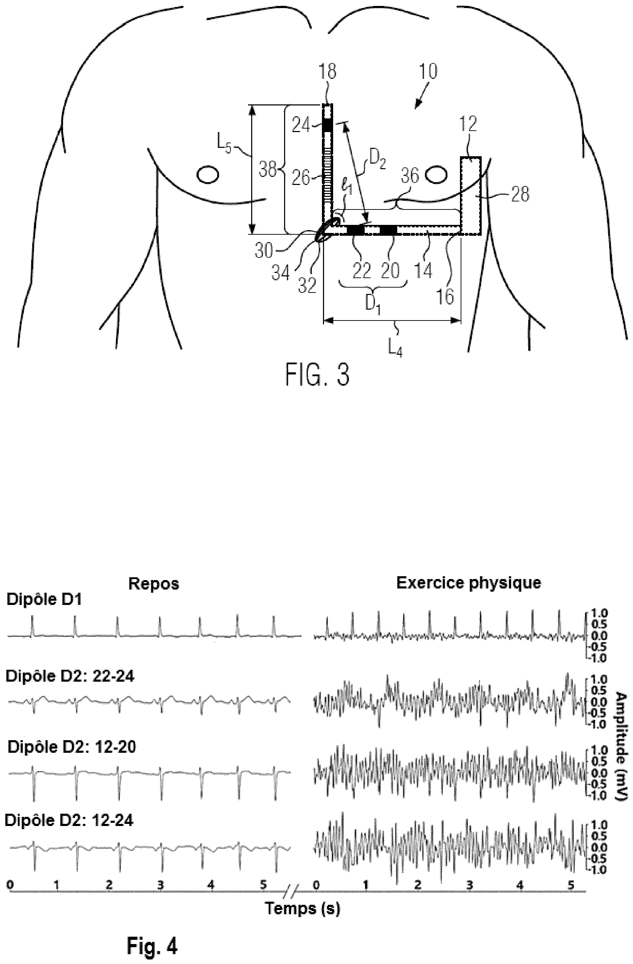

L'invention va maintenant être décrite plus en détail en utilisant des modes de réalisation avantageux d'une manière exemplaire et en référence aux Figures 2 et 3. Les modes de réalisation décrits sont simplement des configurations possibles et il faut garder à l'esprit que les caractéristiques individuelles telles que décrites ci-dessus peuvent être fournies indépendamment les unes des autres ou peuvent être omises tout à fait lors de la mise en oeuvre de la présente invention.

La Figure 2 illustre un système de défibrillation cardiaque implantable sous cutané 10. La Figure 3 illustre ledit système 10 dans un état implanté.

Le système de défibrillation cardiaque implantable sous cutané10 comprend un boîtier 12, générateur d'impulsions, auquel est relié une sonde implantable sous cutanée 14. Le système de défibrillation cardiaque implantable sous cutané 10 est apte à délivrer un choc de défibrillation.

La sonde implantable sous cutanée 14 est au moins partiellement souple et comprend deux extrémités 16, 18 : une extrémité proximale 16 qui est connectée au boîtier 12 et une extrémité distale libre 18.

Dans le mode de réalisation illustrée aux Figures 2 et 3, la sonde implantable sous-cutanée 14 comprend trois électrodes de détection 20, 22, 24 et une électrode de défibrillation 26. Dans une variante, la sonde implantable sous-cutanée 14 pourrait comprendre plus que trois électrodes de détection.

La sonde implantable sous cutanée 14 comprend également des fils conducteurs (qui ne sont pas visibles sur les Figures 2 et 3) permettant de connecter électriquement les électrodes 20, 22, 24 de la sonde 14 à des contacts électriques (non visibles sur les Figures 2 et 3) au niveau du boîtier 12 - connu en soi dans l'état actuel de la technique.

Dans un mode de réalisation avantageux, au moins une des électrodes de détection 20, 22est formée d'un mono-filament (non-représenté) enroulé autour de la sonde implantable sous cutanée 14. Ainsi, dans ce mode de réalisation, au moins une des électrodes de détection 20, 22est une électrode flexible contrairement aux sondes classiques connues qui sont dotées d'anneaux de détection rigides. Les électrodes de détection 20, 22, 24 peuvent être partiellement enrobée d'une sous-couche de silicone ou de polyuréthane.

Les électrodes de détection 20, 22, 24 de la sonde implantable sous-cutanée 14 permettent la détection de signaux électrophysiologiques utilisés pour déduire l'activité cardiaque d'un patient.

La détection d'activité électrophysiologique par voie sous cutanée est cependant altérée par de nombreux artefacts comme des bruits d'origine musculaire ou des interférences avec le milieu extérieur. De plus, la sonde 14 étant de type sous cutanée, les électrodes de détection 20, 22, 24 ne sont pas en contact direct avec le myocarde. La qualité de la détection des signaux électrophysiologiques pour un défibrillateur implantable sous-cutané dépend ainsi largement du positionnement des électrodes de détection 20, 22, 24.

Afin d'améliorer la détection de l'onde R et de minimiser celle des ondes cardiaques P et T, et de réduire le risque de surdétection ou/et de détections erronées, notamment pour faciliter la mesure de l'intervalle R-R, le système de défibrillation cardiaque implantable sous cutané 10 comprend un positionnement spécifique des électrodes de détection 20, 22, 24. Précisément, tel qu'illustré aux Figures 2 et 3, une première électrode de détection 20 et une deuxième électrode de détection 22 sont positionnées entre le boîtier 12 et l'électrode de défibrillation 26 ; tandis qu'une troisième électrode de détection 24 est placée entre l'extrémité distale 18 de la sonde 14 et l'électrode de défibrillation 26. L'électrode de défibrillation 26 est ainsi positionnée entre la deuxième électrode de détection 22 et la troisième électrode de détection 24. Ainsi, dans un sens allant de l'extrémité proximale 16 de la sonde 14 à l'extrémité distale 18 de la sonde 14, la sonde comprend dans cet ordre : la première électrode de détection 20, la deuxième électrode de détection 22, l'électrode de défibrillation 26 puis la troisième électrode de détection 24.

Le positionnement spécifique des électrodes de détection 20, 22, 24 va être décrit en terme de longueur en référence à la Figure 2 seulement, représentant la sonde 14 dans un état non-implanté, non-courbé et aligné selon un axe A.

La première électrode de détection 20 et la deuxième électrode de détection 22 forment un premier dipôle D1 de longueur L1.

Selon le mode de réalisation illustrée par la Figure 2, la deuxième électrode de détection 22 et la troisième électrode de détection 24 forment un deuxième dipôle D2 de longueur L2. Dans une variante, le deuxième dipôle D2 peut être formé par la troisième électrode de détection 24 et la première électrode de détection 20. Dans une autre variante, le deuxième dipôle D2 peut être formé par le boîtier 12 et une des trois électrodes de détection 20, 22, 24.

La longueur L1 du premier dipôle D1 est plus courte que la longueur L2 du deuxième dipôle D2. En particulier, la longueur L1 est compris entre 5 à 50 millimètres, plus en particulier entre 10 et 20 millimètres, tandis que la longueur L2 est comprise entre 50 et 400 millimètres. De plus, la distance L3 entre la première électrode de détection 20 et le boîtier 12 est comprise entre 40 et 300 millimètres.

Le système de défibrillation cardiaque implantable sous cutané10 comprend en outre un contrôleur 28 logé dans le boîtier 12. Le contrôleur 28 du système 10 est configuré pour détecter des signaux électriques enregistrés simultanément via le premier dipôle D1 et le deuxième dipôle D2 de la sonde implantable sous-cutanée 14. Le contrôleur 28 est configuré pour détecter l'onde R d'un signal électrophysiologique au niveau du premier dipôle D1.

Dans un autre mode de réalisation, la détection de l'onde R via le premier dipôle pourrait être combinée à la détection de signaux électrophysiologiques sur une pluralité de « deuxièmes dipôles », c'est-à-dire sur une pluralité de dipôles qui soient plus longs que le premier dipôle, par exemple : un dipôle formé par la deuxième électrode de détection 22 et la troisième électrode de détection 24, un dipôle formé par la première électrode de détection 20 et la troisième électrode de détection 24 et un dipôle formé entre le boîtier 12 et la troisième électrode de détection 24. Dans une variante, le boîtier 12 peut servir d'électrode pour former un deuxième dipôle avec l'une des électrodes de détection 20, 22, 24.

Le premier dipôle D1 étant plus court que le deuxième dipôle D2, le premier dipôle D1 est moins exposé au risque de surdétection, notamment car il est moins sujet à enregistrer des bruits d'origine musculaire. De plus, le premier dipôle D1 est positionné lors de l'implantation sous-cutanée du système 10 proche et au-dessus de l'encoche cardiaque du poumon gauche (en anglais « left lung cardiac notch ») et des ventricules. Cette position particulière du premier dipôle D1 permet de détecter un signal électrophysiologique avec une onde R qui soit davantage distinctif par rapport aux ondes P et T ; les ondes P et T détectées à cet endroit étant minimisées par rapport à l'onde R. Cet effet technique avantageux améliorant la détection de l'onde R via les signaux recueillis à partir du premier dipôle D1 est illustré à la Figure 4.

La Figure 4 représente plusieurs signaux électrophysiologiques d'un patient recueillis dans un état dit au repos et au cours d'un exercice physique, via le dipôle D1 et trois différentes configurations de dipôle D2. Selon une première configuration, le dipôle D2 est formé par la deuxième électrode de détection 22 et la troisième électrode de détection 24 (voir « Dipôle D2 : 22-24 sur la Figure 4). Selon une deuxième configuration, le dipôle D2 est formé par le boiter 12 et la première électrode de détection 20 (voir « Dipôle D2 : 12-20 sur la Figure 4). Selon une troisième configuration, le dipôle D2 est formé par le boîtier 12 et la troisième électrode de détection 24 (voir « Dipôle D2 : 12-24 sur la Figure 4).

Les signaux recueillis en état de repos démontrent la façon dont le dipôle D1 présente des rapports R/T et R/P élevés, c'est-à-dire optimaux, par rapport aux dipôles D2. Le risque de surdétection de l'onde T s'avère donc plus faible sur le dipôle D1. De plus, les signaux recueillis en période d'exercice physique montrent la manière dont des myopotentiels peuvent affecter les signaux. Cependant, les signaux recueillis via le dipôle D1 restent toutefois beaucoup moins affectés que ceux des dipôles D2. Par conséquent, il est plus facile de détecter l'onde R sur le dipôle D1. La raison, déjà évoquée plus haut, vient notamment du fait que le premier dipôle D1 étant plus court que le deuxième dipôle D2, le dipôle D1 est moins sujet à enregistrer des bruits d'origine musculaire, susceptibles d'altérer les signaux.

Afin de permettre de positionner le premier dipôle D1 et le deuxième dipôle D2 par rapport aux tissus du patient, tel qu'illustré à Figure 3, de manière à optimiser la qualité du signal électrophysiologique détecté, la sonde implantable sous-cutanée comprend un moyen de fixation 30.

Le moyen de fixation 30 est positionné entre la deuxième électrode de détection 22 et l'électrode de défibrillation 26. Le moyen de fixation 30 de la sonde implantable sous-cutanée 14 est positionné à une distance L4 comprise entre 60 et 300 millimètres de l'extrémité proximale 16 de la sonde 14 et à une distance L5 comprise entre 50 à 400 millimètres de l'extrémité distale 18 de la sonde 14. Ses dimensions spécifiques L4 et L5 permettent à la fois de définir le positionnement du premier dipôle D1 et du deuxième dipôle D2 l'un par rapport à l'autre, mais aussi par rapport à la masse musculaire du patient lorsque le système 10 est implanté ; le boîtier 12 étant usuellement implanté au même endroit chez chaque patient, c'est-à-dire du côté gauche de la poitrine du patient selon les pratiques médicales connues et tel que représenté sur la Figure 3.

Le moyen de fixation 30 permet en outre d'éviter un déplacement involontaire de la sonde implantable sous-cutanée 14, par exemple généré par un mouvement du patient. Le moyen de fixation 30 facilite ainsi le maintien des positions adéquates du premier dipôle D1 et du deuxième dipôle D2 l'une par rapport à l'autre mais aussi relativement au corps du patient.

Selon un mode de réalisation non limitatif et illustrée à la Figure 2, le moyen de fixation 30 comprend une rainure 32 sur la circonférence de la sonde implantable sous-cutanée 14, la largeur I1 de la rainure 32 étant dimensionnée pour y loger un fil de ligature 34. Un praticien peut ainsi attacher la sonde implantable sous-cutanée 14 aux tissus, par exemple les tissus musculaires, d'un patient en réalisant une ligature sous-cutanée au niveau du moyen de fixation 30.

Le positionnement du moyen de fixation 30 sur la sonde implantable sous-cutanée 14 permet également de définir la position du changement de courbe de la sonde implantable sous-cutanée 14. En effet, quand elle est implantée de manière sous-cutanée, la sonde implantable sous-cutanée 14 est courbée au niveau du moyen de fixation 30 (voir Figure 3), de sorte qu'une première portion 36 de la sonde 14 entre l'extrémité proximale 16 et le moyen de fixation 30 soit essentiellement perpendiculaire à une deuxième portion 38 de la sonde 14 entre l'extrémité distale 18 et le moyen de fixation 30. Ainsi, le premier dipôle D1 et le deuxième dipôle D2 sont préférentiellement arrangeables de manière à ne pas être alignés entre eux. Il s'avère que cet agencement essentiellement perpendiculaire entre la première portion 36 de la sonde et la deuxième portion 38 de la sonde 14, réalisée au niveau du moyen de fixation 30, permet d'améliorer davantage la qualité des signaux détectés, notamment car la perpendicularité des portions 36, 38 de la sonde 14 implantée permet de détecter des signaux dans un repère quasi orthogonal. Le fait que les signaux détectés via le premier dipôle et via le deuxième dipôle soient recueillis de manière quasi orthogonale permet de fournir deux perspectives différentes des signaux électrophysiologiques, desquelles davantage d'informations peuvent être extraites et recoupées entre elles ; que depuis une seule perspective. Ces informations peuvent servir à identifier le rythme cardiaque d'un patient.

Dans un autre mode de réalisation, le système de défibrillation cardiaque implantable sous cutané 10 peut en outre comprendre un accéléromètre ou/et un gyroscope configuré pour détecter la position du patient. De ce fait, en permettant la détection de la position du patient, l'interprétation des signaux électrophysiologiques détectés peut être davantage affinée, et donc améliorée.

Dans un autre mode de réalisation, la sonde implantable sous cutanée 14 peut en outre comprendre un moyen isolant déplaçable sur la première portion 36 de la sonde 14 de manière à couvrir au moins partiellement le premier dipôle D1 - permettant ainsi de décaler la position du dipôle D1 sur la première portion 36 de la sonde 14.

Ainsi, les dimensions spécifiques L1, L2 L3, L4 et L5 caractérisant le système de défibrillation cardiaque implantable sous cutané 10, la sélection particulière des dipôles et la détection des ondes R enregistrées via le premier dipôle permettent d'améliorer la qualité de la détection de l'onde R du système 10 par rapport aux systèmes de défibrillation cardiaque implantables sous cutanés connus.

Dans un autre mode de réalisation, la détection de l'onde R via le premier dipôle pourrait être combinée à la détection de signaux électrophysiologiques sur une pluralité de « deuxièmes dipôles », c'est-à-dire sur une pluralité de dipôles qui soient plus longs que le premier dipôle, par exemple : un dipôle formé par la deuxième électrode de détection 22 et la troisième électrode de détection 24, un dipôle formé par la première électrode de détection 20 et la troisième électrode de détection 24 et un dipôle formé entre le boîtier 12 et l'une des électrodes de détection 20, 22, 24.

Les modes de réalisation décrits sont simplement des configurations possibles et il faut garder à l'esprit que les caractéristiques individuelles des différents modes de réalisation peuvent être combinées entre elles ou fournies indépendamment les unes des autres.

## Revendications

1. Un système de défibrillation cardiaque implantable sous cutané comprenant :
un boîtier (12); et
une sonde implantable sous cutanée (14) comprenant deux extrémités (16, 18), une extrémité proximale (16) étant connectée au boîtier (12) et une extrémité distale (18) étant une extrémité libre (18) ;
la sonde implantable sous cutanée (14) comprenant au moins une électrode de défibrillation (26) et au moins trois électrodes de détection (20, 22, 24) ;
la première électrode de détection (20) et la deuxième électrode de détection (22) formant un premier dipôle (D1) ;
la troisième électrode de détection (24) et la première électrode de détection (20), ou, la troisième électrode de détection (24) et la deuxième électrode de détection (22), ou, le boîtier (12) et l'une desdites électrodes de détection (20, 22, 24), formant un deuxième dipôle (D2);
l'électrode de défibrillation (26) étant positionnée entre la deuxième électrode de détection (22) et la troisième électrode de détection (24);
le premier dipôle (D1) étant positionné entre le boîtier (12) et l'électrode de défibrillation (26);
la troisième électrode (24) étant positionnée entre l'extrémité distale libre (18) de la sonde (14) et l'électrode de défibrillation (26); et
la longueur (L1) du premier dipôle (D1) étant plus courte que la longueur (L2) du deuxième dipôle (D2).

2. Le système de défibrillation cardiaque implantable sous cutané selon la revendication 1, comprenant en outre un contrôleur (28) configuré pour la détection de signaux électriques concurremment enregistrés via le premier dipôle (D1) et le deuxième dipôle (D2) de la sonde implantable sous cutanée (14) ;
le contrôleur (28) étant configuré pour la détection de l'onde R d'un signal électrique via le premier dipôle (D1).

3. Le système de défibrillation cardiaque implantable sous cutané selon l'une des revendications précédentes, dont la longueur (L1) du premier dipôle (D1), soit la distance entre la première électrode de détection (20) et la deuxième électrode de détection (22), est comprise entre 5 à 50 millimètres, en particulier entre 10 et 20 millimètres ; et
dont la longueur (L2) du deuxième dipôle (D2), soit la distance entre la troisième électrode de détection (24) et la première électrode de détection (20), ou, la troisième électrode de détection (24) et la deuxième électrode de détection (22), ou, le boîtier (12) et l'une desdites électrodes de détection (20, 22, 24), est comprise entre 50 et 400 millimètres.

4. Le système de défibrillation cardiaque implantable sous cutané selon l'une des revendications précédentes, dont la sonde implantable sous cutanée (14) comprend au moins un moyen de fixation (30) pour fixer la sonde implantable sous cutanée (14) aux tissus d'un patient.

5. Le système de défibrillation cardiaque implantable sous cutané selon la revendication 4, dont le au moins un moyen de fixation (30) de la sonde implantable sous cutanée (14) est positionné entre la deuxième électrode de détection (22) et l'électrode de défibrillation (26).

6. Le système de défibrillation cardiaque implantable sous cutané selon la revendication 5, dont le au moins un moyen de fixation (30) de la sonde implantable sous cutanée (14) est positionné entre 60 et 300 millimètres de l'extrémité proximale (16); et
le au moins un moyen de fixation (30) de la sonde implantable sous cutanée (14) est positionné entre 50 et 400 millimètres de l'extrémité distale (18).

7. Le système de défibrillation cardiaque implantable sous cutané selon l'une des revendications 5 à 6, dont le au moins un moyen de fixation (30) de la sonde implantable sous cutanée (14) est une rainure (32) sur la circonférence de la sonde implantable sous cutanée (14), la largeur de la rainure (32) étant dimensionnée pour y loger un fil de ligature (34).

8. Le système de défibrillation cardiaque implantable sous cutané selon l'une des revendications précédentes, dont la distance (L3) entre le boîtier (12) et la première électrode détection (20) est comprise entre 40 et 300 millimètres.

9. Le système de défibrillation cardiaque implantable sous cutané selon l'une des revendications précédentes, comprenant en outre un accéléromètre ou/et un gyroscope configuré(s) pour détecter la position du patient.

10. Le système de défibrillation cardiaque implantable sous cutané selon l'une des revendications précédentes, dont le deuxième dipôle (D2) est formé par la troisième électrode de détection (24) et la deuxième électrode de détection (22).

## Patentansprüche

1. Implantierbares subkutanes Herzdefibrillationssystem mit:
einem Gehäuse (12); und
einer subkutan implantierbaren Sonde (14) mit zwei Enden (16, 18), die ein mit dem Gehäuse verbundenes proximales Ende (16) und ein als freies Ende (18) gebildetes distales Ende (18) aufweist;
wobei die subkutan implantierbare Sonde (14) mindestens eine Defibrillationselektrode (26) und mindestens drei Detektionselektroden (20, 22, 24) aufweist;
wobei die erste Detektionselektrode (20) und die zweite Detektionselektrode (22) einen ersten Dipol (D1) bilden;
wobei die dritte Detektionselektrode (24) und die erste Detektionselektrode (20) oder die dritte Detektionselektrode (24) und die zweite Detektionselektrode (22), oder das Gehäuse (12) und eine der Detektionselektroden (20, 22, 24) einen zweiten Dipol (D2) bilden;
wobei die Defibrillationselektrode (26) zwischen der zweiten Detektionselektrode und der dritten Detektionselektrode (24) angeordnet ist;
wobei der erste Dipol (D1) zwischen dem Gehäuse (12) und der Defibrillationselektrode (26) angeordnet ist;
wobei die dritte Elektrode (24) zwischen dem freien distalen Ende (18) der Sonde (14) und der Defibrillationselektrode (26) angeordnet ist; und
wobei die Länge (L1) des ersten Dipols (D1) kürzer ist als die Länge (L2) des zweiten Dipols (D2).

2. Implantierbares subkutanes Herzdefibrillationssystem nach Anspruch 1, ferner umfassend eine Steuerung (28) ausgestaltet zur Erkennung von gleichzeitig über den ersten Dipol (D1) und den zweiten Dipol (D2) der subkutan implantierbaren Sonde (14) erfassten elektrischen Signalen;
wobei die Steuerung (28) zur Erfassung der R-Welle eines elektrischen Signals über den ersten Dipol (D1) ausgestaltet ist.

3. Implantierbares subkutanes Herzdefibrillationssystem nach einem der vorhergehende Ansprüche, wobei die Länge (L1) des ersten Dipols (D1), also der Abstand zwischen der ersten Detektionselektrode (20) und der zweiten Detektionselektrode (22), zwischen 5 und 50 Millimeter, insbesondere zwischen 10 und 20 Millimeter, beträgt; und
wobei die Länge (L2) des zweiten Dipols (D2), also der Abstand zwischen der dritten Detektionselektrode (24) und der ersten Detektionselektrode (20) oder der dritten Detektionselektrode (24) und der zweiten Detektionselektrode (22) oder dem Gehäuse (12) und einer der Detektionselektroden (20, 22, 24), zwischen 50 und 400 Millimeter beträgt.

4. Implantierbares subkutanes Herzdefibrillationssystem nach einem der vorhergehende Ansprüche, wobei die subkutan implantierbare Sonde (14) mindestens ein Befestigungsmittel (30) zum Befestigen der subkutan implantierbaren Sonde (14) am Gewebe eines Patienten aufweist.

5. Implantierbares subkutanes Herzdefibrillationssystem nach Anspruch 4, wobei das mindestens eine Befestigungsmittel (30) der subkutan implantierbaren Sonde (14) zwischen der zweiten Detektionselektrode (22) und der Defibrillationselektrode (26) angeordnet ist.

6. Implantierbares subkutanes Herzdefibrillationssystem nach Anspruch 5, wobei das mindestens eine Befestigungsmittel (30) der subkutan implantierbaren Sonde (14) zwischen 60 und 300 Millimeter von dem proximalen Ende (16) entfernt angeordnet ist; und
das mindestens eine Befestigungsmittel (30) der subkutan implantierbaren Sonde (14) zwischen 50 und 400 Millimeter von dem distalen Ende (18) entfernt angeordnet ist.

7. Implantierbares subkutanes Herzdefibrillationssystem nach einem der Ansprüche 5 bis 6, wobei das mindestens eine Befestigungsmittel (30) der subkutan implantierbaren Sonde (14) eine Nut (32) am Umfang der subkutan implantierbaren Sonde (14) ist, wobei die Breite der Nut (32) so dimensioniert ist, dass dort ein Ligaturdraht (34) untergebracht werden kann.

8. Implantierbares subkutanes Herzdefibrillationssystem nach einem der vorhergehenden Ansprüche, wobei der Abstand (L3) zwischen dem Gehäuse (12) und der ersten Detektionselektrode (20) zwischen 40 und 300 Millimeter beträgt.

9. Implantierbares subkutanes Herzdefibrillationssystem nach einem der vorhergehenden Ansprüche, ferner umfassend einen Beschleunigungssensor und/oder ein Gyroskop, ausgestaltet zum Erkennen der Patientenposition.

10. Implantierbares subkutanes Herzdefibrillationssystem nach einem der vorhergehenden Ansprüche, wobei der zweite Dipol (D2) durch die dritte Detektionselektrode (24) und die zweite Detektionselektrode (22) gebildet ist.

## Claims

1. A subcutaneous implantable cardiac defibrillation system comprising:
a housing (12); and
a subcutaneous implantable lead (14) comprising two ends (16, 18), a proximal end (16) being connected to the housing (12) and a distal end (18) being a free end (18);
the subcutaneous implantable lead (14) comprising at least one defibrillating electrode (26) and at least three sensing electrodes (20, 22, 24);
the first sensing electrode (20) and the second sensing electrode (22) forming a first dipole (D1);
the third sensing electrode (24) and the first sensing electrode (20), or the third sensing electrode (24) and the second sensing electrode (22), or the housing (12) and one of said sensing electrodes (20, 22, 24), forming a second dipole (D2);
the defibrillating electrode (26) being positioned between the second sensing electrode (22) and the third sensing electrode (24);
the first dipole (D1) being positioned between the housing (12) and the defibrillating electrode (26);
the third electrode (24) being positioned between the free distal end (18) of the lead (14) and the defibrillating electrode (26); and
the length (L1) of the first dipole (D1) being shorter than the length (L2) of the second dipole (D2).

2. The subcutaneous implantable cardiac defibrillation system according to claim 1, further comprising a controller (28) configured to sense electrical signals being concurrently recorded via the first dipole (D1) and the second dipole (D2) of the subcutaneous implantable lead (14),
the controller (28) being configured to sense the R wave of an electrical signal via the first dipole (D1).

3. The subcutaneous implantable cardiac defibrillation system according to one of the preceding claims, wherein the length (L1) of the first dipole (D1), that is the distance between the first sensing electrode (20) and the second sensing electrode (22), is in a range of 5 to 50 millimeters, in particular of 10 to 20 millimeters; and
wherein the length (L2) of the second dipole (D2), that is the distance between the third sensing electrode (24) and the first sensing electrode (20), or between the third sensing electrode (24) and the second sensing electrode (22), or between the housing (12) and one of said sensing electrodes (20, 22, 24), is in a range of 50 to 400 millimeters.

4. The subcutaneous implantable cardiac defibrillation system according to one of the preceding claims, wherein the subcutaneous implantable lead (14) comprises at least one securing means (30) for securing the subcutaneous implantable lead (14) to a patient's tissues.

5. The subcutaneous implantable cardiac defibrillation system according to claim 4, wherein the at least one securing means (30) of the subcutaneous implantable lead (14) is positioned between the second sensing electrode (22) and the defibrillating electrode (26).

6. The subcutaneous implantable cardiac defibrillation system according to claim 5, wherein the at least one securing means (30) of the subcutaneous implantable lead (14) is positioned between 60 and 300 millimeters from the proximal end (16); and
the at least one securing means (30) of the subcutaneous implantable lead (14) is positioned between 50 and 400 millimeters from the distal end (18).

7. The subcutaneous implantable cardiac defibrillation system according to one of the claims 5 to 6, wherein the at least one securing means (30) of the subcutaneous implantable lead (14) is a groove (32) on the circumference of the subcutaneous implantable lead (14), the width of the groove (32) being sized to accommodate a ligature wire (34).

8. The subcutaneous implantable cardiac defibrillation system according to one of the preceding claims, wherein the distance (L3) between the housing (12) and the first sensing electrode (20) is in a range of 40 to 300 millimeters.

9. The subcutaneous implantable cardiac defibrillation system according to one of the preceding claims, further comprising an accelerometer or/and a gyroscope configured to sense the patient's position.

10. The subcutaneous implantable cardiac defibrillation system according to one of the preceding claims, wherein the second dipole (D2) is formed by the third sensing electrode (24) and the second sensing electrode (22).
